# EUROPEAN PATENT APPLICATION

(11) **EP 4 372 080 A1**
(43) Date of publication of application: **22.05.2024**
(21) Application number: 22842176.4
(22) Date of filing: 14.07.2022
(51) Int. Cl.: C12N 5/071, A61K 35/44, A61K 35/545, A61P 9/00, A61P 9/10, A61P 43/00

(54) **PERICYTE-LIKE CELL EXPRESSING VASCULAR ENDOTHELIAL GROWTH FACTOR (VEGF) AT HIGH LEVEL**

(30) Priority: 15.07.2021 JP 2021116869
(71) Applicant: Astellas Pharma Inc., Chuo-ku Tokyo 103-8411 (JP); OSAKA UNIVERSITY, Suita-shi Osaka 565-0871 (JP); National Center for Child Health and Development, Tokyo 157-8535 (JP)
(72) Inventor: SHIMATANI, Kenichiro, Tokyo 103-8411 (JP); SATO, Hiromu, Tokyo 103-8411 (JP); SASAI, Masao, Suita-shi, Osaka 565-0871 (JP); SAWA, Yoshiki, Suita-shi, Osaka 565-0871 (JP); SAITO, Atsuhiro, Suita-shi, Osaka 565-0871 (JP); MIYAGAWA, Shigeru, Suita-shi, Osaka 565-0871 (JP)
(74) Representative: Hoffmann Eitle
(86) International application number: PCT/JP2022/027696
(87) International publication number: WO 2023/286834

(57) **Abstract**

An object of the present invention is to provide a pericyte-like cell having high angiogenic potential with a higher cell proliferation ability than a primary pericyte available in the past and high VEGF expression, and a method for producing the same. Provided are a method for producing a VEGF-highly expressing pericyte-like cell, the method including selecting a CD56(-) pericyte-like cell from a population including a pericyte-like cell obtained by inducing differentiation of a pluripotent stem cell; and a VEGF-highly expressing pericyte-like cell produced by the production method.

## Description

### TECHNICAL FIELD

The present invention relates to a method for producing a vascular endothelial growth factor (VEGF)-highly expressing pericyte-like cell, a method for selecting a population of VEGF-highly expressing pericyte-like cells, an angiogenic therapy including administering the VEGF-highly expressing pericyte-like cell, a VEGF-highly expressing pericyte-like cell produced by the production method, a population of VEGF-highly expressing pericyte-like cells selected by the selection method, a pharmaceutical composition including the VEGF-highly expressing pericyte-like cell, and the like.

### BACKGROUND ART

A capillary connects an arteriole and a venule, and is distributed deep in the body tissue in a networked pattern so that oxygen and a nutrient can be supplied to every corner of a periphery of the body. The capillary is composed of a single layer of a vascular endothelial cell that forms a luminal structure; and a pericyte (vascular pericyte) surrounding the same. The pericyte, as a cell that covers a vascular endothelial cell, plays an important role in normal blood flow regulation such as maturing and stabilizing a blood vessel and maintaining the blood-brain barrier. There are critical lower limb ischemia and intractable ulcer as a serious disease that impairs blood flow, no effective drug therapy for these has been established, and the disease is treated by bypass surgery, endovascular treatment, or the like. In recent years, there has been a demand for the development of a new therapeutic method that induces the neogenesis of peripheral capillaries by a cell therapy (PTL 1).

An embryonic stem cell (ES cell), an induced pluripotent stem cell (iPS cell) obtained by introducing a reprogramming factor into a somatic cell, and the like have been reported as cells having pluripotency. The development of an angiogenic therapy for a peripheral vascular disease represented by critical lower limb ischemia, in which a pericyte-like cell or the like obtained by inducing differentiation of such a pluripotent stem cell is transplanted, has been studied (NPL 1). The use of a pericyte-like cell or the like obtained by inducing differentiation of a pluripotent stem cell is expected to provide a cell of interest as a therapeutic agent with no lot-to-lot differences.

As a method for inducing differentiation of a human pluripotent stem cell into a pericyte-like cell, a method via an embryoid body (EB) has been studied (NPL 1). A pericyte-like cell induced from a human pluripotent stem cell via an EB cooperates with a vascular endothelial cell and shows the formation of a blood vessel in an angiogenic plug model, but the poor proliferation ability of the pericyte-like cell poses a difficult challenge to obtain a large number of cells as a source of cell therapy (NPL 1). On the other hand, a method for inducing differentiation from a human pluripotent stem cell into a pericyte-like cell via early mesoderm (primitive mesoderm) has also been reported (PTL 2). In this method, an early mesodermal cell is added to a viscous methylcellulose medium and allowed to form the cell mass (spheroid) under the methylcellulose medium over a period of about 2 weeks. The resulting cell mass is then cultured in a monolayer to differentiate into pericyte-like cells. The differentiation into a pericyte-like cell can be confirmed by using a surface antigen marker such as NG2 or CD146 (NPL 2).

The VEGF is a glycoprotein involved in vasculogenesis during embryogenesis and angiogenesis in the living body and mainly binds to a vascular endothelial growth factor receptor (VEGF receptor) on the surface of a vascular endothelial cell to stimulate cell division, migration, and differentiation of the vascular endothelial cell. In addition, it also has an action of enhancing vascular permeability in a microvessel.

As described above, a pericyte plays an important role in the maturation and stabilization of a blood vessel, one of the mechanisms is considered to be secretion of VEGF by the pericyte. That is, VEGF acts on survival and stabilization of a vascular endothelial cell in a juxtacrine (contact secretion) or paracrine (paracrine secretion) manner through the production of VEGF by a pericyte (NPL 3).

### CITATION LIST

### PATENT LITERATURE

PTL 1: WO2008/104064
PTL 1: US9,868,939

### NON PATENT LITERATURE

NPL 1: Dar A et al., Circulation, (2012), 125: 87-99
NPL 2: Birbrair A et al., Stem Cell Research, (2013), 10: 67-84
NPL 3: Darland DC et al., Dev. Biol., (2003), 264: 275-288

### SUMMARY OF INVENTION

### TECHNICAL PROBLEM

An object of the present invention is to provide a pericyte-like cell having high angiogenic potential with a higher cell proliferation ability than a primary pericyte available in the past and high VEGF expression, and a method for producing the same.

### SOLUTION TO PROBLEM

In order to solve the above problem, the present inventors have carried out intensive studies and found that among pericyte-like cells obtained by inducing differentiation of a pluripotent stem cell, the expression level of VEGF is significantly higher in a pericyte-like cell negative for the expression of CD56 (CD56(-) pericyte-like cell) than in a pericyte-like cell positive for the expression of the cell marker CD56 (CD56(+) pericyte-like cell). They have also found that such a CD56(-) pericyte-like cell has a significantly higher cell proliferation ability than a primary pericyte derived from a living body and that a CD56(-) pericyte-like cell transplanted into the living body has significantly higher angiogenic potential than a CD56(+) pericyte-like cell. The present invention has been completed based on such findings.

That is, the present invention has the following features:
[1] A method for producing a VEGF-highly expressing pericyte-like cell, the method including a step of selecting a CD56(-) pericyte-like cell from a population including a pericyte-like cell obtained by inducing differentiation of a pluripotent stem cell.
[2] The production method according to [1], including the following steps before the step of selecting a CD56(-) pericyte-like cell from a population including a pericyte-like cell:
   (a) a step of differentiating a pluripotent stem cell into an early mesodermal cell; and
   (b) a step of differentiating the early mesodermal cell obtained in step (a) into a pericyte-like cell.
[3] The production method according to [2], wherein step (b) includes the following steps:
   (b-1) a step of forming a spheroid of the early mesodermal cell obtained in step (a); and
   (b-2) a step of differentiating the early mesodermal cell forming the spheroid into a pericyte-like cell.
[4] The production method according to [3], wherein step (b-1) of forming a spheroid is performed in a culture vessel with a plurality of mortar-shaped fine wells on a culture surface without a gap.
[5] The production method according to any one of [1] to [4], wherein the pluripotent stem cell is a human pluripotent stem cell.
[6] The production method according to any one of [1] to [5], wherein the pluripotent stem cell is an embryonic stem cell (ES cell) or an induced pluripotent stem cell (iPS cell).
[7] A method for selecting a population of VEGF-highly expressing pericyte-like cells using a cell surface marker of CD56(-) from a population including a pericyte-like cell obtained by inducing differentiation of a pluripotent stem cell.
[8] The production method according to [7], wherein the population including a pericyte-like cell is induced by a method including the following steps:
   (a) a step of differentiating a pluripotent stem cell into an early mesodermal cell; and
   (b) a step of differentiating the early mesodermal cell obtained in step (a) into a pericyte-like cell.
[9] The method according to [8], wherein step (b) includes the following steps:
   (b-1) a step of forming a spheroid of the early mesodermal cell obtained in step (a); and
   (b-2) a step of differentiating the early mesodermal cell forming the spheroid into a pericyte-like cell.
[10] The production method according to [9], wherein step (b-1) of forming a spheroid is performed in a culture vessel with a plurality of mortar-shaped fine wells on a culture surface without a gap.
[11] The production method according to any one of [7] to [10], wherein the pluripotent stem cell is a human pluripotent stem cell.
[12] The method according to any one of [7] to [11], wherein the pluripotent stem cell is an ES cell or an iPS cell.
[13] An angiogenic therapy including administering a therapeutically effective amount of a VEGF-highly expressing pericyte-like cell produced by the production method according to any one of [1] to [6] to a subject.
[14] The angiogenic therapy according to [13], wherein the angiogenic therapy further includes administering a vascular endothelial cell.
[15] The angiogenic therapy according to [13] or [14], wherein the angiogenic therapy is a treatment for critical lower limb ischemia.
[16] Use of a VEGF-highly expressing pericyte-like cell produced by the production method according to any one of [1] to [6] in production of a pharmaceutical composition for an angiogenic therapy.
[17] Use of a VEGF-highly expressing pericyte-like cell produced by the production method according to any one of [1] to [6] in production of a pharmaceutical composition for treatment for critical lower limb ischemia.
[18] A VEGF-highly expressing pericyte-like cell obtained by a production method including a step of selecting a CD56(-) pericyte-like cell from a population including a pericyte-like cell obtained by inducing differentiation of a pluripotent stem cell.
[19] The VEGF-highly expressing pericyte-like cell according to [18], obtained by a production method including (a) a step of differentiating a pluripotent stem cell into an early mesodermal cell;
   (b) a step of differentiating the early mesodermal cell obtained in step (a) into a pericyte-like cell; and
   (c) a step of selecting a CD56(-) pericyte-like cell from a population including the pericyte-like cell.
[20] The VEGF-highly expressing pericyte-like cell according to [18], obtained by a production method including (a) a step of differentiating a pluripotent stem cell into an early mesodermal cell;
   (b-1) a step of forming a spheroid of the early mesodermal cell obtained in step (a);
   (b-2) a step of differentiating the early mesodermal cell forming the spheroid into a pericyte-like cell; and
   (c) a step of selecting a CD56(-) pericyte-like cell from a population including the pericyte-like cell.
[21] The VEGF-highly expressing pericyte-like cell according to [18], obtained by a production method including (a) a step of differentiating a pluripotent stem cell into an early mesodermal cell;
   (b-1') a step of forming a spheroid of the early mesodermal cell obtained in step (a) in a culture vessel comprising a plurality of mortar-shaped fine wells on a culture surface without a gap;
   (b-2) a step of differentiating the early mesodermal cell forming the spheroid into a pericyte-like cell; and
   (c) a step of selecting a CD56(-) pericyte-like cell from a population including the pericyte-like cell.
[22] The VEGF-highly expressing pericyte-like cell according to any one of [18] to [21], wherein the pluripotent stem cell is a human pluripotent stem cell.
[23] The VEGF-highly expressing pericyte-like cells according to any one of [18] to [22], wherein the pluripotent stem cell is an ES cell or an iPS cell.
[24] A population of VEGF-highly expressing pericyte-like cells, which is selected using a cell surface marker of CD56(-) from a population including a pericyte-like cell obtained by inducing differentiation of a pluripotent stem cell.
[25] The population of VEGF-highly expressing pericyte-like cells according to [24], which is selected using a cell surface marker of CD56(-) from a population including a pericyte-like cell induced to differentiate by a method including (a) a step of differentiating a pluripotent stem cell into an early mesodermal cell; and (b) a step of differentiating the early mesodermal cell obtained in step (a) into a pericyte-like.
[26] The population of VEGF-highly expressing pericyte-like cells according to [24], which is selected using a cell surface marker of CD56(-) from a population including a pericyte-like cell induced to differentiate by a method including (a) a step of differentiating a pluripotent stem cell into an early mesodermal cell;
   (b-1) a step of forming a spheroid of the early mesodermal cell obtained in step (a); and
   (b-2) a step of differentiating the early mesodermal cell forming the spheroid into a pericyte-like cell.
[27] The population of VEGF-highly expressing pericyte-like cells according to [24], which is selected using a cell surface marker of CD56(-) from a population including a pericyte-like cell induced to differentiate by a method including (a) a step of differentiating a pluripotent stem cell into an early mesodermal cell;
   (b-1') a step of forming a spheroid of the early mesodermal cell obtained in step (a) in a culture vessel comprising a plurality of mortar-shaped fine wells on a culture surface without a gap; and
   (b-2) a step of differentiating the early mesodermal cell forming the spheroid into a pericyte-like cell.
[28] The population of VEGF-highly expressing pericyte-like cells according to any one of [24] to [27], wherein the pluripotent stem cell is a human pluripotent stem cell.
[29] The population of VEGF-highly expressing pericyte-like cells according to any one of [24] to [28], wherein the pluripotent stem cell is an ES cell or an iPS cell.
[30] A pharmaceutical composition for an angiogenic therapy, including the VEGF-highly expressing pericyte-like cell according to any one of 18] to [23].
[31] The pharmaceutical composition according to [30], wherein the angiogenic therapy is a treatment for critical lower limb ischemia.
[32] The pharmaceutical composition according to [30] or [31], wherein the pharmaceutical composition is used in combination with a vascular endothelial cell.
[33] A pharmaceutical composition for an angiogenic therapy, including a combination of the VEGF-highly expressing pericyte-like cell according to any one of [18] to [23] and a vascular endothelial cell.
[34] The pharmaceutical composition according to [33], wherein the angiogenic therapy is a treatment for critical lower limb ischemia.
[35] The VEGF-highly expressing pericyte-like cell according to any one of [18] to [23] for use in an angiogenic therapy.
[36] The VEGF-highly expressing pericyte-like cell according to any one of [18] to [23] for use in treatment for critical lower limb ischemia.
[37] An angiogenic therapy including administering a therapeutically effective amount of a population of VEGF-highly expressing pericyte-like cells selected by the method according to any one of [7] to [12] to a subject.
[38] The angiogenic therapy according to [37], wherein the angiogenic therapy further includes administering a vascular endothelial cell.
[39] The angiogenic therapy according to [37] or [38], wherein the angiogenic therapy is a treatment for critical lower limb ischemia.
[40] Use of a population of VEGF-highly expressing pericyte-like cells selected by the method according to any one of [7] to [12] in production of a pharmaceutical composition for an angiogenic therapy.
[41] Use of a population of VEGF-highly expressing pericyte-like cells selected by the method according to any one of [7] to [12] in production of a pharmaceutical composition for treatment for critical lower limb ischemia.
[42] A pharmaceutical composition for an angiogenic therapy, including the population of VEGF-highly expressing pericyte-like cells according to any one of [24] to [29].
[43] The pharmaceutical composition according to [42], wherein the angiogenic therapy is a treatment for critical lower limb ischemia.
[44] The pharmaceutical composition according to [42] or [43], wherein the pharmaceutical composition is used in combination with a vascular endothelial cell.
[45] A pharmaceutical composition for an angiogenic therapy, including a combination of the population of VEGF-highly expressing pericyte-like cells according to any one of [24] to [29] and a vascular endothelial cell.
[46] The pharmaceutical composition according to [45], wherein the angiogenic therapy is a treatment for critical lower limb ischemia.
[47] The population of VEGF-highly expressing pericyte-like cells according to any one of [24] to [29] for use in an angiogenic therapy.
[48] The population of VEGF-highly expressing pericyte-like cells according to any one of [24] to [29] for use in treatment for critical lower limb ischemia.

### ADVANTAGEOUS EFFECTS OF INVENTION

In the present invention, by selecting a CD56(-) pericyte-like cell from a population including a pericyte-like cell obtained by inducing differentiation of a pluripotent stem cell, a pericyte-like cell highly expressing VEGF can be obtained and produced. In addition, a CD56(-) pericyte-like cell obtained by the method of the present invention can be used for an angiogenic therapy for critical lower limb ischemia or the like.

### BRIEF DESCRIPTION OF DRAWINGS

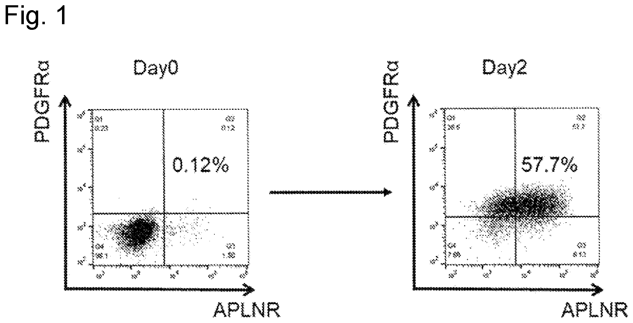

[Fig. 1] Fig. 1 is a diagram confirming differentiation of a human ES cell into an early mesodermal cell after 48 hours (2 days) of culture of the human ES cell in STEMdiff Mesoderm Induction Medium (STEMCELL Technologies, 05220). The differentiation into the early mesodermal cell was confirmed by flow cytometry using an increase in expression of PDGFR α and APLNR, which are cell surface markers of an early mesodermal cell, as an indicator. The vertical axis in Fig. 1 represents the PDGFRα expression level, and the horizontal axis represents the APLNR expression level. The left diagram and the right diagram of Fig, 1 each show the expression levels of PDGFRα and APLNR on the cell surface before culture (Day 0) and after 48 hours of culture (Day 2), respectively.

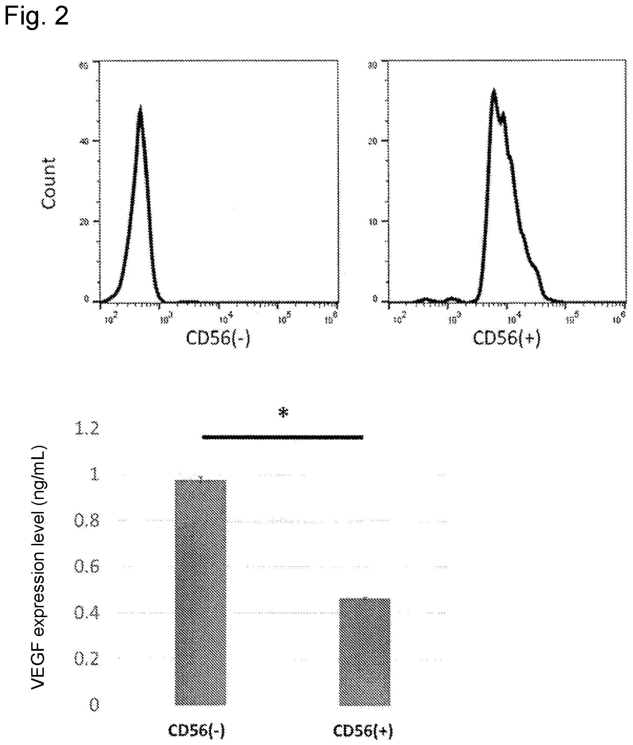

[Fig. 2] The upper diagram of Fig. 2 shows results of selecting and separating pericyte-like cells obtained by inducing differentiation of a spheroid of the early mesodermal cell in Example 3 as a CD56(-) pericyte-like cell (labeled CD56(-)) and a CD56(+) pericyte-like cell (labeled CD56(+)) in Example 4. The vertical axis in the upper diagram represents the number of cells (Count), and the horizontal axis represents the intensity of cell surface expression of CD56. The lower diagram of Fig. 2 shows results of evaluating VEGF expression levels in each of the CD56(-) pericyte-like cell and the CD56(+) pericyte-like separated in Example 4, in Example 5. The vertical axis in the lower diagram represents the VEGF expression level (µg/mL). The P value represented by * in the lower diagram is less than 5% (p < 0.05). An error bar represents ± standard error of the mean.

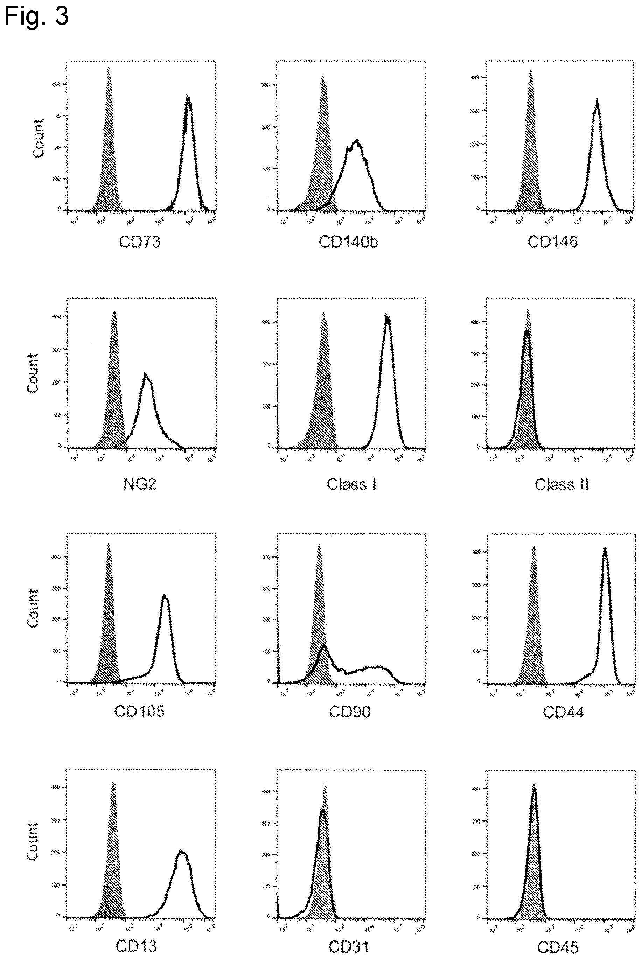

[Fig. 3] Fig. 3 shows results of evaluating the expression characteristics of CD73, CD105, CD 140b, CD90, CD146, CD44, NG2, CD13, HLA-ABC (Class I), CD31, HLA-DR,DP,DQ (Class II), and CD45, and the respective cell surface antigens in CD56(-) pericyte-like cells separated in Example 4, in Example 6. Gray areas show results with isotype antibodies and black lines with evaluation antibodies. The vertical axis in Fig. 3 represents the number of cells (Count), and the horizontal axis represents the signal intensity of each cell surface antigen.

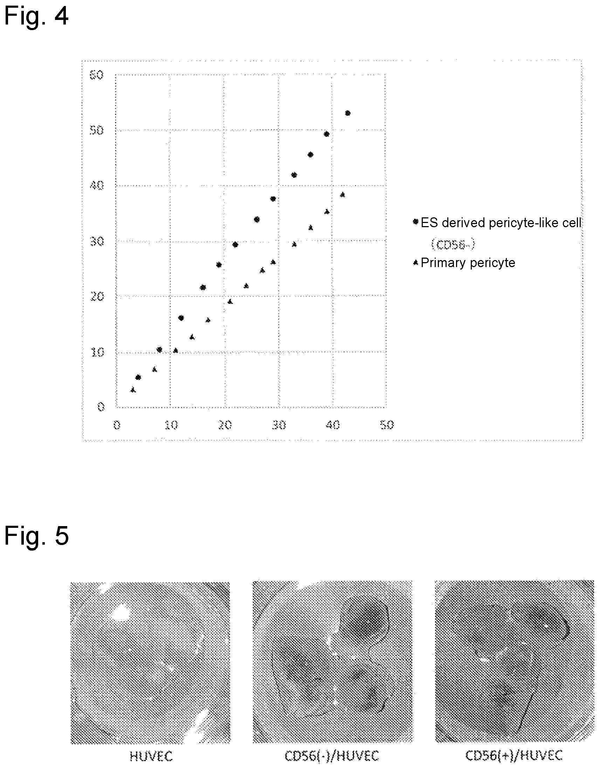

[Fig. 4] Fig. 4 shows results of comparing the cell proliferation rate of the CD56(-) pericyte-like cell selected in Example 4 with that of a primary pericyte in Example 7. The vertical axis in Fig. 4 represents the cumulative number of cell divisions, and the horizontal axis represents the number of passage days.
[Fig. 5] Fig, 5 shows results of qualitatively evaluating the angiogenic potential of the CD56(-) pericyte-like cell in Example 8. Specifically, Fig. 5 shows results of subcutaneously administering a human umbilical vein endothelial cell (HUVEC) alone (labeled HUVEC), HUVEC and a CD56(-) pericyte-like cell (labeled CD56(-)/HUVEC), or HUVEC and a CD56(+) pericyte-like cell (labeled CD56(+)/HUVEC) to an NOG mouse together with Matrigel, and recovering Matrigel 14 days later and photographing the same.

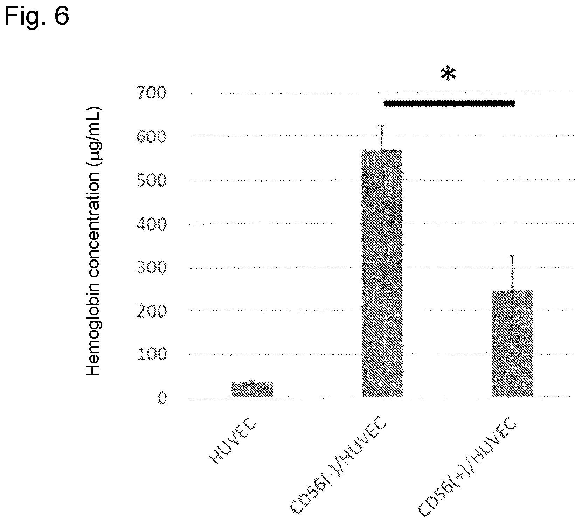

[Fig. 6] Fig. 6 shows results of quantitatively evaluating the angiogenic potential of the CD56(-) pericyte-like cell in Example 9. Specifically, Fig. 6 shows results of crushing each Matrigel including CD56(-)/HUVEC, CD56(+)/HUVEC, and HUVEC alone, which is recovered by the procedure of Example 8 and measuring the hemoglobin concentration of the supernatant after centrifugation. The vertical axis in Fig. 6 represents the hemoglobin concentration (g/mL) of the Matrigel-derived supernatant. The P value represented by * in Fig. 6 is less than 5% (p < 0.05). An error bar represents ± standard error of the mean.

### DESCRIPTION OF EMBODIMENTS

The present invention will be described in detail below.

### <Method for producing VEGF-highly expressing pericyte-like cell and method for selecting population of VEGF-highly expressing pericyte-like cell>

The present invention provides a method for producing a VEGF-highly expressing pericyte-like cell (hereinafter also referred to as the "production method of the present invention"), the method including a step of selecting a CD56(-) pericyte-like cell from a population including a pericyte-like cell obtained by inducing differentiation of a pluripotent stem cell.

The present invention also provides a method for selecting a population of VEGF-highly expressing pericyte-like cells using a cell surface marker of CD56(-) from a population including a pericyte-like cell obtained by inducing differentiation of a pluripotent stem cell (hereinafter also referred to as the "selection method of the present invention").

### «Population including pericyte-like cell obtained by inducing differentiation of pluripotent stem cell»

The term "pericyte" refers to a cell present in the brain, the periphery, the retina, or the like in such a way as to surround a microvascular wall or a capillary wall, and is also referred to as a vascular pericyte. The function thereof is as described above, and the pericyte, as a cell that covers a vascular endothelial cell, plays an important role in normal blood flow regulation such as maturating and stabilizing a blood vessel and maintaining the blood-brain barrier (Daneman R et al., Nature, (2010), 468: 562-568; Armulik A et al., Dev. Cell, (2011), 21: 193-215). When a cell function of a pericyte is impaired, an original function of the pericyte is impaired, leading to a serious blood flow disorder-related disease such as diabetic retinopathy. In addition, among skeletal muscle-derived pericytes, the presence of a cell referred to as a mesoangioblast, which has the ability to differentiate into a muscle or a bone, has been clarified. (Gerli MFM et al., J. Vis. Exp., (2014), 83: 50523, Gerli MFM et al., Stem Cell Rep., (2019), 12: 461-473, Shimatani K et al., Am. J. Physiol. Heart Circ. Physiol., (2021), on line: https://doi.org/10.1152/ajpheart.00470.2020).

### -Pericyte-like cell-

As used herein, the term "pericyte-like cell" means a cell that is obtained by inducing differentiation of a pluripotent stem cell and has properties similar to those of a primary pericyte (see below). It can be confirmed by a known method that a pericyte-like cell has properties similar to those of a pericyte (Armulik A et al., Dev. Cell, (2011), 21: 193-215). The induction of differentiation of a pluripotent stem cell into a pericyte-like cell can be carried out by using a method known to those skilled in the art (WO2013/108039, WO2009/156151, US9771561, US9868939, US2015/0368609, US2017/0342384, US2019/0316094). For example, the induction of differentiation of a pluripotent stem cell into a pericyte-like cell can be carried out by using the method described in the section [Method for inducing differentiation of pluripotent stem cell into pericyte-like cell] described later.

The term "primary pericyte" means a pericyte directly collected from an individual organism, or a primary cultured cell or a subcultured cell obtained by culturing and proliferating the pericyte in vitro. A method for isolating a primary pericyte from an individual organism and a method for culturing the same are disclosed in, for example, Quattrocelli M et al., Methods Mol. Biol., (2012), 798: 65-76.

### -Pluripotent stem cell-

As used herein, the term "pluripotent stem cell" means a stem cell that has pluripotency by which the cell can differentiate into many cells having different properties and morphologies that are present in the living body, and that also has proliferative potential. A preferred pluripotent stem cell in the present invention is a human pluripotent stem cell. In one embodiment, the population including a pericyte-like cell obtained by inducing differentiation of a pluripotent stem cell used in the production method of the present invention and the selection method of the present invention is a population including a pericyte-like cell obtained by inducing differentiation of a human pluripotent stem cell.

The pluripotent stem cell used in the present invention is not particularly limited, and examples thereof include an embryonic stem cell (ES cell), an embryonic stem cell prepared by using a nuclear transfer technique (nuclear transfer embryonic stem cell; ntES cell), a sperm stem cell (germline stem cell; GS cell), an embryonic germ cell (EG cell), an induced pluripotent stem cell (iPS cell), and a pluripotent cell derived from a cultured fibroblast or bone marrow stem cell (multi-lineage differentiating stress enduring cell; Muse cell). A preferred pluripotent stem cell for inducing differentiation into a pericyte-like cell in the present invention is an ES cell or an iPS cell. In one embodiment, the population including a pericyte-like cell obtained by inducing differentiation of a pluripotent stem cell used in the production method of the present invention and the selection method of the present invention is a population including a pericyte-like cell obtained by inducing differentiation of a ES cell or a iPS cell. In one embodiment, the population including a pericyte-like cell obtained by inducing differentiation of a pluripotent stem cell used in the production method of the present invention and the selection method of the present invention is a population including a pericyte-like cell obtained by inducing differentiation of a human ES cell or a human iPS cell.

### -ES cell-

An ES cell is a stem cell that is established from the inner cell mass of an early embryo (for example, a blastocyst) of a mammal such as a human or a mouse and that has pluripotency and proliferative potential through self-renewal.

The ES cell can be established by taking out the inner cell mass from the blastocyst of a fertilized egg of a target animal and culturing the inner cell mass on a fibroblast feeder. In addition, the maintenance of a cell by subculture can be carried out by using a medium supplemented with a substance such as leukemia inhibitory factor (LIF) or basic fibroblast growth factor (basic FGF; bFGF). A human ES cell can be established and maintained by a known method (disclosed in, for example, Suemori H et al., Biochem. Biophys. Res. Commun., (2006), 345: 926-932, or Kawasaki H et al., Proc. Natl. Acad. Sci. USA, (2002), 99: 1580-1585).

### -iPS cell-

An iPS cell is a general term for a pluripotent stem cell line that is artificially induced by introducing a specific gene into a somatic cell having lost the pluripotency thereof. A method for producing an iPS cell is known in the art and may be produced by introducing a reprogramming factor into an arbitrary somatic cell. Here, examples of the reprogramming factor include a gene or a gene product such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, or Glis1, and these reprogramming factors may be used singly, or a combination thereof may be used. Examples of the combination of the reprogramming factors include the combinations disclosed in WO2007/069666; WO2008/118820; WO2009/007852;WO2009/032194; WO2009/058413; WO2009/057831; WO2009/075119; WO2009/079007;WO2009/091659; WO2009/101084; WO2009/101407; WO2009/102983; WO2009/114949; WO2009/117439; WO2009/126250; WO2009/126251; WO2009/126655; WO2009/157593; WO2010/009015; WO2010/033906; WO2010/033920; WO2010/042800; WO2010/050626; WO 2010/056831; WO2010/068955; WO2010/098419; WO2010/102267; WO2010/111409; WO2010/111422; WO2010/115050; WO2010/124290; WO2010/147395; WO2010/147612; Huangfu D et al., Nat. Biotechnol., (2008), 26: 795-797; Shi Yet al., Cell Stem Cell, (2008), 2: 525-528; Eminli S et al., Stem Cells, (2008), 26: 2467-2474; Huangfu D et al., Nat. Biotechnol., (2008), 26: 1269-1275; Shi Y et al., Cell Stem Cell, (2008), 3: 568-574; Zhao Y et al., Cell Stem Cell, (2008), 3: 475-479; Marson A Cell Stem Cell, (2008), 3: 132-135; Feng B et al., Nat. Cell Biol., (2009), 11: 197-203; Judson RL et al., Nat. Biotechnol., (2009), 27: 459-461; Lyssiotis CA et al., Proc. Natl. Acad. Sci. USA, (2009), 106: 8912-8917; Kim JB et al., Nature, (2009), 461: 649-643; Ichida JK et al., Cell Stem Cell, (2009), 5: 491-503; Heng JC et al., Cell Stem Cell, (2010), 6: 167-174; Han J et al., Nature, (2010), 463: 1096-1100; Mali P et al., Stem Cells, (2010), 28: 713-720; Maekawa M et al., Nature, (2011), 474: 225-229.

The somatic cell used for the production of an iPS cell may be either of a somatic cell of a neonate and a somatic cell of a healthy human or a patient, and is not particularly limited to these. In addition, any of a primary cultured cell, a passaged cell, and an established cell derived therefrom may be used. In an embodiment, examples of the somatic cell used for the production of an iPS cell include (1) a tissue stem cell (somatic stem cell) such as a neural stem cell, a hematopoietic stem cell, a mesenchymal stem cell, or a dental pulp stem cell, (2) a tissue progenitor cell, and (3) a differentiated cell present in an organ or a tissue such as a blood cell (peripheral blood cell, umbilical cord blood cell, or the like), a muscle cell, a skin cell, a hair cell, a liver cell, a gastric mucosa cell, an enterocyte, a splenic cell, a pancreatic cell, a brain cell, a lung cell, a kidney cell, and an adipocyte.

When an iPS cell is used as a material for inducing differentiation into a pericyte-like cell, it is desirable to use an iPS cell of an individual who is a transplant recipient having the same or substantially the same human leukocyte antigen (HLA) genotype from the viewpoint of avoiding rejection. Here, the term "substantially the same" means that the HLA genotypes match each other to the extent that an immune reaction to the transplanted cell can be suppressed by an immunosuppressive agent, and for example, such a cell is an iPS cell derived from a somatic cell having an HLA type in which 3 loci of HLA-A, HLA-B, and HLA-DR or 4 loci consisting of these 3 loci and HLA-C are matched.

As a pluripotent stem cell, which is a material for inducing a pericyte-like cell, a pluripotent stem cell that does not cause a rejection reaction in allotransplantation prepared by the method disclosed in, for example, Gornalusse GG et al., Nat. Biotechnol., (2017), 35: 765-772 can also be used. The pluripotent stem cell that does not cause a rejection reaction in allotransplantation is preferably an ES cell or an iPS cell that does not cause a rejection reaction in allotransplantation, and more preferably a human ES cell or a human iPS cell that does not cause a rejection reaction in allotransplantation. In one embodiment, the population including a pericyte-like cell obtained by inducing differentiation of a pluripotent stem cell used in the production method of the present invention and the selection method of the present invention is a population including a pericyte-like cell obtained by inducing differentiation of a pluripotent stem cell that does not cause a rejection reaction in allotransplantation. In one embodiment, the population including a pericyte-like cell obtained by inducing differentiation of a pluripotent stem cell used in the production method of the present invention and the selection method of the present invention is a population including a pericyte-like cell obtained by inducing differentiation of a human ES cell or a human iPS cell that does not cause a rejection reaction in allotransplantation.

### [Method for inducing differentiation of pluripotent stem cell into pericyte-like cell]

In the present invention, a pericyte-like cell can be obtained by inducing differentiation of a pluripotent stem cell into a pericyte-like cell, as described above. The method for inducing differentiation of a pluripotent stem cell into a pericyte-like cell is not particularly limited, and in one embodiment, a pericyte-like cell can be obtained by a method including the following steps (a) and (b):
(a) a step of differentiating a pluripotent stem cell into an early mesodermal cell; and
(b) a step of differentiating the early mesodermal cell obtained in step (a) into a pericyte-like cell.

### Steps (a) and (b) will be described below.

Step (a) is a step of differentiating a pluripotent stem cell into an early mesodermal cell. In the present invention, the method for differentiating a pluripotent stem cell into an early mesodermal cell is not particularly limited, and the method for inducing differentiation of a pluripotent stem cell into an early mesodermal cell disclosed in, for example, PTL 2, US9,771,561, or Uenishi G et al., Stem Cell Reports, (2014), 3: 1073-1084 can be used. In one embodiment, the pluripotent stem cell can be differentiated into an early mesodermal cell by culturing a pluripotent stem cell in STEMdiff Mesoderm Induction Medium, as described in Example 1. In the present invention, the differentiation of a pluripotent stem cell into an early mesodermal cell can be confirmed by using a surface antigen marker (PDGFRα, APLNR, or the like) specific to an early mesodermal cell as disclosed in, for example, Vodyanik MA et al., Cell Stem Cell, (2010), 7: 718-729, US9,771,561, and Uenishi G et al., Stem Cell Reports, (2014), 3: 1073-1084.

Step (b) is a step of inducing differentiation of the early mesodermal cell obtained in the step (a) into a pericyte-like cell. In the present invention, the method for differentiating the early mesodermal cell into a pericyte-like cell is not particularly limited, and the method for inducing differentiation of an early mesodermal cell into a pericyte-like cell disclosed in, for example, PTL 2 can be used. In one embodiment, an early mesodermal cell is cultured on a fibronectin- and collagen-coated plate by using serum-free medium including bFGF and platelet-derived growth factor-BB (PDGF-BB) to differentiate into a pericyte-like cell. In one embodiment, a spheroid of the early mesodermal cell is formed, as described below, the early mesodermal cell forming a spheroid is cultured on a fibronectin- and collagen-coated plate by using a serum-free medium including bFGF and PDGF-BB to differentiate into a pericyte-like cell. The differentiation of the early mesodermal cell into a pericyte-like cell can be confirmed by using a surface antigen marker such as NG2 or CD146 (Herrmann M et al., Eur. Cells Mater., (2016), 31: 236-249, Covas DT et al., Exp. Hematol., (2008), 36: 642-654, Lv FJ et al., Stem Cells, (2014), 32: 1408-1419).

In one embodiment, step (b) includes the following steps:
(b-1) a step of forming a spheroid of the early mesodermal cell obtained in step (a); and
(b-2) a step of differentiating the early mesodermal cell forming the spheroid into a pericyte-like cell.

In the present invention, the method for forming a spheroid of the early mesodermal cell is not particularly limited, and a known method can be used. Examples thereof include the method using a methylcellulose medium disclosed in US9,771,561. In one embodiment, a spheroid of an early mesodermal cell can be formed by culturing the early mesodermal cell on a spheroid-forming vessel with a spheroid formation medium, as described in Example 2. The composition of a spheroid formation medium can be set with reference to a known technique (Vodyanik MA et al., Cell Stem Cell, (2010), 7: 718-729, or the like). In one embodiment of step (b-1), an early mesodermal cell is cultured in a semisolid medium including bFGF (for example, a methylcellulose medium) to form a spheroid of the early mesodermal cells.

The shape of the spheroid-forming vessel in which spheroid formation is carried out is also not particularly limited. In one embodiment, step (b-1) of forming a spheroid is carried out in a culture vessel with a plurality of mortar-shaped fine wells on a culture surface without a gap. For example, a commercially available dish used in Example 2 can be used as the culture vessel with a plurality of mortar-shaped fine wells on the culture surface without a gap.

### <<Using cell surface marker of CD56(-) to select CD56(-) pericyte-like cell>>

In the production method of the present invention, "a CD56(-) pericyte-like cell is selected" from a population including a pericyte-like cell. In addition, in the selection method of the present invention, "a cell surface marker of CD56(-) is used" in the selection of a population of VEGF-highly expressing pericyte-like cells from a population including a pericyte-like cell. The term "using a cell-surface marker of CD56 (-)" in the selection method of the present invention has the same meaning as "selecting a pericyte-like cell of CD56(-)" in the production method of the present invention, and these are hereinafter collectively referred to as the "selection step of the present invention". The selection of the present invention is used for selecting a VEGF-highly expressing pericyte-like cell or a population of the VEGF-highly expressing pericyte-like cell.

As used herein, the labeling of CD56(+) means that cells are positive for the cell-surface antigen CD56. As used herein, the labeling of CD56(-) means that cells are negative for CD56. A cell being negative for a cell surface antigen means that there is no expression of the cell surface antigen or that the expression level of the cell surface antigen is very low.

### -CD56-

CD56 (GeneID: 4684), also called neural cell adhesion molecule 1 (NCAM1), NCAM, or MSK39, is a member of the immunoglobulin superfamily and a glycoprotein that functions as a cell adhesion molecule. CD56 is involved in embryogenesis, development and interactions through contacts between neurons. There are multiple isoforms in human CD56. (Lanier LL et al., J. Immunol., (1991), 146: 4421-4426, van Duijnhoven HLP et al., Int. J. Cancer, (1992), 50: 118-123, Rossel RJ et al., Biochim. Biophys. Acta, (1992), 1130: 95-96). For example, the NCBI accession number for the type 1 isoform of the human CD56 protein is NP_000606.3.

CD56 is expressed on an NK cell and some T cells in the hematopoietic system and on a neuron, glia, a Schwann cell, or the like in the nervous system. CD56 is also expressed in a skeletal muscle cell and is further expressed in multiple tumor cells (Van Acker HH et al., Front. Immunol., (2017), 8: Art. 892). On the other hand, in the isolation of a primary pericyte, one of the indicators is that the cell is CD56(-) (Billaud M et al., Stem Cell Rep., (2017), 9: 292-303).

In one embodiment, the CD56 used in the selection step of the present invention is human CD56. Several antibodies specific for human CD56 are commercially available. One example of such antibodies includes the anti-human CD56 antibody used in Example 4.

In the selection step of the present invention, selection of a pericyte-like cell that is CD56(-) or use of a cell-surface marker of CD56(-) can be carried out by various methods known in the art. Examples thereof include a method of using an antibody that specifically binds to CD56 to select a CD56(-) cell based on the strength of binding of the anti-CD56 antibody to the cell. Such a method includes the use of a fluorescently labeled antibody and a fluorescence-activated cell sorter (FACS).

### <VEGF-highly expressing pericyte-like cell or a population of VEGF-highly expressing pericyte-like cell>

The present invention provides a VEGF-highly expressing pericyte-like cells (hereinafter also referred to as the "cell of the present invention") obtained by a production method including a step of selecting a CD56(-) pericyte-like cell from a population including a pericyte-like cell obtained by inducing differentiation of a pluripotent stem cell.

The present invention provides a population of VEGF-highly expressing pericyte-like cells (hereinafter also referred to as the "cell population of the present invention") selected using a cell surface marker of CD56(-) from a population including a pericyte-like cell obtained by inducing differentiation of a pluripotent stem cell.

The cell of the present invention and a cell in the cell population of the present invention each have a feature of high expression of VEGF. High expression of VEGF by the cell of the present invention and the cell in the cell population of the present invention means significantly higher expression of VEGF when compared to a pericyte-like cell selected using a cell surface marker of CD56(+) from a population including a pericyte-like cell obtained by inducing differentiation of a pluripotent stem cell.

In one embodiment, the cell of the present invention is a VEGF-highly expressing pericyte-like cell obtained by a production method including (a) a step of differentiating a pluripotent stem cell into an early mesodermal cell; (b) a step of differentiating the early mesodermal cell obtained in step (a) into a pericyte-like cell; and (c) a step of selecting a CD56(-) pericyte-like cell from a population including the pericyte-like cell. In one embodiment, the cell of the present invention is a VEGF-highly expressing pericyte-like cell obtained by a production method including (a) a step of differentiating a pluripotent stem cell into an early mesodermal cell; (b-1) a step of forming a spheroid of the early mesodermal cell obtained in step (a); (b-2) a step of differentiating the early mesodermal cell forming the spheroid into a pericyte-like cell; and (c) a step of selecting a CD56(-) pericyte-like cell from a population including the pericyte-like cell. In one embodiment, the cell of the present invention is a VEGF-highly expressing pericyte-like cell obtained by a production method including (a) a step of differentiating a pluripotent stem cell into an early mesodermal cell; (b-1') a step of forming a spheroid of the early mesodermal cell obtained in step (a) in a culture vessel comprising a plurality of mortar-shaped fine wells on a culture surface without a gap; (b-2) a step of differentiating the early mesodermal cell forming the spheroid into a pericyte-like cell; and (c) a step of selecting a CD56(-) pericyte-like cell from a population including the pericyte-like cell.

In one embodiment, the cell of the present invention is a VEGF-highly expressing pericyte-like cell obtained by a production method including a step of selecting a CD56(-) pericyte-like cell from a population including a pericyte-like cell obtained by inducing differentiation of a human pluripotent stem cell. In one embodiment, the cell of the present invention is a VEGF-highly expressing pericyte-like cell obtained by a production method including a step of selecting a CD56(-) pericyte-like cell from a population including a pericyte-like cell obtained by inducing differentiation of an ES cell or an iPS cell. In one embodiment, the cell of the present invention is a VEGF-highly expressing pericyte-like cell obtained by a production method including a step of selecting a CD56(-) pericyte-like cell from a population including a pericyte-like cell obtained by inducing differentiation of a human ES cell or a human iPS cell.

In one embodiment, the cell population of the present invention is a population of VEGF-highly expressing pericyte-like cells, which is selected using a cell surface marker of CD56(-) from a population including a pericyte-like cell induced to differentiate by a method including (a) a step of differentiating a pluripotent stem cell into an early mesodermal cell; and (b) a step of differentiating the early mesodermal cell obtained in step (a) into a pericyte-like. In one embodiment, the cell population of the present invention is a population of VEGF-highly expressing pericyte-like cells, which is selected using a cell surface marker of CD56(-) from a population including a pericyte-like cell induced to differentiate by a method including (a) a step of differentiating a pluripotent stem cell into an early mesodermal cell; (b-1) a step of forming a spheroid of the early mesodermal cell obtained in step (a); and (b-2) a step of differentiating the early mesodermal cell forming the spheroid into a pericyte-like cell. In one embodiment, the cell population of the present invention is a population of VEGF-highly expressing pericyte-like cells, which is selected using a cell surface marker of CD56(-) from a population including a pericyte-like cell induced to differentiate by a method including (a) a step of differentiating a pluripotent stem cell into an early mesodermal cell; (b-1') a step of forming a spheroid of the early mesodermal cell obtained in step (a) in a culture vessel comprising a plurality of mortar-shaped fine wells on a culture surface without a gap; and (b-2) a step of differentiating the early mesodermal cell forming the spheroid into a pericyte-like cell.

In one embodiment, the cell population of the present invention is a population of VEGF-highly expressing pericyte-like cells, which is selected using a cell surface marker of CD56(-) from a population including a pericyte-like cell obtained by inducing differentiation of a human pluripotent stem cell. In one embodiment, the cell population of the present invention is a population of VEGF-highly expressing pericyte-like cells, which is selected using a cell surface marker of CD56(-) from a population including a pericyte-like cell obtained by inducing differentiation of an ES cell or an iPS cell. In one embodiment, the cell population of the present invention is a population of VEGF-highly expressing pericyte-like cells, which is selected using a cell surface marker of CD56(-) from a population including a pericyte-like cell obtained by inducing differentiation of a human ES cell or a human iPS cell.

### [Method for culturing pericyte-like cell]

The method for culturing and proliferating the cell of the present invention and the cell population of the present invention is not particularly limited, and a method for culturing and proliferating a pericyte known in the art can be used.

The medium for culturing the cell of the present invention and the cell population of the present invention is not particularly limited as long as it is a medium suitable for culturing a pericyte-like cell, and MEM medium, BME medium, D-MEM medium, **α**-MEM medium, IMEM medium, ES medium, DM-160 medium, Fisher medium, F12 medium, WE medium, RPMI medium, StemSpan medium, StemPro medium, and a mixture thereof can be used as a basal medium.

The medium for culturing the cell of the present invention and the cell population of the present invention may be appropriately supplemented with various nutrient sources necessary for cell maintenance and proliferation. The medium can include, for example as a nutrient source, a carbon source such as glycerol, glucose, fructose, sucrose, lactose, honey, starch, or dextrin; a hydrocarbon such as fatty acid, oil/fat, lecithin, or an alcohol; a nitrogen source such as ammonium sulfate, ammonium nitrate, ammonium chloride, urea, or sodium nitrate; an inorganic salt such as common salt, a potassium salt, a phosphate, a magnesium salt, a calcium salt, an iron salt, or a manganese salt; monopotassium phosphate, dipotassium phosphate, magnesium sulfate, sodium chloride, ferrous sulfate, sodium molybdate, sodium tungstate, and manganese sulfate; various vitamins; an amino acid; or the like. In one embodiment, an amino acid such as glutamine can be used as a nutrient source added to the medium for culturing the cell of the present invention and the cell population of the present invention.

The medium for culturing the cell of the present invention and the cell population of the present invention may be appropriately supplemented with a growth factor such as an FGF family growth factor necessary for cell proliferation. The growth factor to be added is not particularly limited, and in one embodiment, bFGF or modified bFGF having enhanced thermostability can be used as a growth factor to be added to the medium for culturing the cell of the present invention and the cell population of the present invention.

The pH of the medium for culturing the cell of the present invention and the cell population of the present invention is in the range of 5.5 to 9.0, preferably 6.0 to 8.0, and more preferably 6.5 to 7.5.

The pericyte-like cell is an adherent cell that has the property of adhering to an extracellular matrix to proliferate. Because of this, in one embodiment, a suitable scaffold can be used in culturing the cell of the present invention and the cell population of the present invention. The scaffold is not particularly limited as long as it is a matrix, a substrate, or a carrier to which a cell can adhere to divide and proliferate, and examples thereof include fibronectin, vitronectin, collagen, proteoglycan, laminin, tenascin, entactin, elastin, fibrillin, hyaluronic acid, gelatin, poly-L-lysine, and poly-D-lysine. In one embodiment, a collagen matrix can be used as a scaffold for use in culturing the cell of the present invention and the cell population of the present invention.

In one embodiment, the culture of the cell of the present invention and the cell population of the present invention can be carried out at 36°C to 38°C. In one embodiment, the culture of the cell of the present invention and the cell population of the present invention can be carried out at 36.5°C to 37.5°C in an atmosphere of 1% to 25% O₂ and 1% to 15% CO₂ while appropriately exchanging the medium.

### <Pharmaceutical composition and the like of the present invention>

The present invention also provides a pharmaceutical composition including the cell of the present invention or the cell population of the present invention (hereinafter collectively referred to as the "cell or the like of the present invention" throughout this section) (also referred to as the "pharmaceutical composition of the present invention"). The pharmaceutical composition can be prepared by a commonly used method by using an excipient commonly used in the art, that is, a pharmaceutical excipient, a pharmaceutical carrier, or the like. In formulating the pharmaceutical composition, an excipient, a carrier, an additive, or the like according to the dosage form therefor can be used in a pharmaceutically acceptable range. In one embodiment, the pharmaceutical composition of the present invention includes a VEGF-highly expressing pericyte-like cell obtained by a production method including a step of selecting a CD56(-) pericyte-like cell from a population including a pericyte-like cell obtained by inducing differentiation of a pluripotent stem cell. In one embodiment, the pharmaceutical composition of the present invention includes a VEGF-highly expressing pericyte-like cell obtained by a production method including (a) a step of differentiating a pluripotent stem cell into an early mesodermal cell; (b) a step of differentiating the early mesodermal cell obtained in step (a) into a pericyte-like cell; and (c) a step of selecting a CD56(-) pericyte-like cell from a population including the pericyte-like cell. In one embodiment, the pharmaceutical composition of the present invention includes a VEGF-highly expressing pericyte-like cell obtained by a production method including (a) a step of differentiating a pluripotent stem cell into an early mesodermal cell; (b-1) a step of forming a spheroid of the early mesodermal cell obtained in step (a); (b-2) a step of differentiating the early mesodermal cell forming the spheroid into a pericyte-like cell; and (c) a step of selecting a CD56(-) pericyte-like cell from a population including the pericyte-like cell. In one embodiment, the pharmaceutical composition of the present invention includes a VEGF-highly expressing pericyte-like cell obtained by a production method including (a) a step of differentiating a pluripotent stem cell into an early mesodermal cell; (b-1') a step of forming a spheroid of the early mesodermal cell obtained in step (a) in a culture vessel comprising a plurality of mortar-shaped fine wells on a culture surface without a gap; (b-2) a step of differentiating the early mesodermal cell forming the spheroid into a pericyte-like cell; and (c) a step of selecting a CD56(-) pericyte-like cell from a population including the pericyte-like cell.

In one embodiment, the pharmaceutical composition of the present invention includes a VEGF-highly expressing pericyte-like cell obtained by a production method including a step of selecting a CD56(-) pericyte-like cell from a population including a pericyte-like cell obtained by inducing differentiation of a human pluripotent stem cell. In one embodiment, the pharmaceutical composition of the present invention includes a VEGF-highly expressing pericyte-like cell obtained by a production method including a step of selecting a CD56(-) pericyte-like cell from a population including a pericyte-like cell obtained by inducing differentiation of an ES cell or an iPS cell. In one embodiment, the pharmaceutical composition of the present invention includes a VEGF-highly expressing pericyte-like cell obtained by a production method including a step of selecting a CD56(-) pericyte-like cell from a population including a pericyte-like cell obtained by inducing differentiation of a human ES cell or a human iPS cell.

In one embodiment, the pharmaceutical composition of the present invention is a pharmaceutical composition for an angiogenic therapy. The pharmaceutical composition encompasses a therapeutic agent for an angiogenic therapy including the cell or the like of the present invention. The angiogenic therapy is a treatment method that, in order to increase the amount of oxygen-rich blood reaching an organ, a tissue, or a site of the body that is in an ischemic state, promotes the formation of a new blood vessel in the organ, tissue, or site of the body that is in an ischemic state. The angiogenic therapy includes a treatment for a peripheral vascular disease such as critical lower limb ischemia or diabetic retinopathy, or a disease such as pulmonary hypertension or intractable ulcer. In one embodiment, the pharmaceutical composition of the present invention is a pharmaceutical composition for treatment for a peripheral vascular disease such as critical lower limb ischemia or diabetic retinopathy, pulmonary hypertension, intractable ulcer, or the like. In one embodiment, the pharmaceutical composition of the present invention is a pharmaceutical composition for treatment for critical lower limb ischemia.

The present invention provides use of the cell or the like of the present invention in production of a pharmaceutical composition for an angiogenic therapy. The present invention provides the cell or the like of the present invention for use in an angiogenic therapy. In addition, the present invention provides use of the cell or the like of the present invention for an angiogenic therapy.

The present invention provides use of the cell or the like of the present invention in production of a pharmaceutical composition for treating critical lower limb ischemia. The present invention provides the cell or the like of the present invention for use in treatment for critical lower limb ischemia. In addition, the present invention provides use of the cell or the like of the present invention for treatment for critical lower limb ischemia.

The present invention also provides an angiogenic therapy including administering a therapeutically effective amount of the cell or the like of the present invention to a subject (also referred to as "the treatment method of the present invention"). In the treatment method of the present invention, the "subject" is a human or a non-human animal in need of treatment. In one embodiment, the "subject" is a human in need of the treatment method. When the cell or the like of the present invention is administered to a human, it can be administered to the subject in the form of a pharmaceutical composition including the cell or the like of the present invention and a pharmaceutically acceptable excipient. The dosage and frequency of administration of the pharmaceutical composition of the present invention to a human can be appropriately regulated according to the disease to be treated, the severity thereof, the age, body weight, and condition of the human to be treated, and the like.

In one embodiment, the treatment method of the present invention is an angiogenic therapy for treatment for critical lower limb ischemia.

The method for administering the pharmaceutical composition of the present invention is not particularly limited, and depending on the site of application, local transplantation by surgical means, intravenous administration, lower limb puncture administration, local infusion administration, subcutaneous administration, intradermal administration, intramuscular administration, or the like can be considered.

The pharmaceutical composition of the present invention may be formed into a sheet and applied directly to an affected area. The sheet may include not only a cell but also a suitable support.

The pharmaceutical composition of the present invention may include a scaffold material or a component that assists in cell maintenance or proliferation, administration to an affected area, and a different pharmaceutically acceptable carrier. Examples of the component necessary for cell maintenance or proliferation include a medium component such as a carbon source, a nitrogen source, a vitamin, a mineral, a salt, or various cytokines, and an extracellular matrix preparation such as Matrigel.

The cell or the like of the present invention or the pharmaceutical composition of the present invention can also be used in combination with a vascular endothelial cell. As used herein, the term "use in combination" means administering a plurality of active pharmaceutical ingredients simultaneously or separately to the same subject. For use in combination, the plurality of active pharmaceutical ingredients may be included in the same composition, or may be included separately in different compositions. In one embodiment, the pharmaceutical composition of the present invention is a pharmaceutical composition used in combination with a vascular endothelial cell. In one embodiment, the pharmaceutical composition of the present invention is a pharmaceutical composition further including a vascular endothelial cell. In one embodiment, the treatment method of the present invention further includes administering a vascular endothelial cell.

The present invention also includes a pharmaceutical composition including a combination of the cell or the like of the present invention and a vascular endothelial cell. As used herein, the term "including a combination of" means that a plurality of active pharmaceutical ingredients are included in the same pharmaceutical composition, or a plurality of active pharmaceutical ingredients are included separately in different pharmaceutical compositions. In one embodiment, the pharmaceutical composition including a combination of the cell or the like of the present invention and a vascular endothelial cell is a pharmaceutical composition including the cell or the like of the present invention and a vascular endothelial cell. In one embodiment, the pharmaceutical composition including a combination of the cell or the like of the present invention and a vascular endothelial cell is a combination of pharmaceutical compositions in which the cell or the like of the present invention and a vascular endothelial cell are separately included in different pharmaceutical compositions. In one embodiment, the pharmaceutical composition including a combination of the cell or the like of the present invention and a vascular endothelial cell is a combination of a pharmaceutical composition including the cell or the like of the present invention and a pharmaceutical composition including a vascular endothelial cell. In one embodiment, the pharmaceutical composition including a combination of the cell or the like of the present invention and a vascular endothelial cell is a pharmaceutical composition for an angiogenic therapy. In one embodiment, the pharmaceutical composition including a combination of the cell or the like of the present invention and a vascular endothelial cell is a pharmaceutical composition for treating critical lower limb ischemia.

### «Vascular endothelial cell»

A "vascular endothelial cell" is one layer of a flat cell that lines the vascular lumen, and has various functions such as regulation of vascular tonicity and vascular permeability, angiogenesis, and promotion of blood coagulation. A large blood vessel at the level of an artery or a vein has a three-layer structure composed of the intima, the media, and the adventitia, each of which is mainly composed of a vascular endothelial cell, a smooth muscle cell, and a fibroblast. On the other hand, in a small blood vessel at the capillary level, the luminal structure of a vascular endothelial cell is surrounded by pericytes. In a mature capillary, a pericyte shares the basement membrane with a vascular endothelial cell and is present in the form of being embedded therein. In recent years, it has been known that a pericyte and a vascular endothelial cell carry out cell signaling mutually to regulate differentiation and proliferation and play an important role in the maturation, stabilization, and maintenance of a capillary, formation of the basement membrane, deposition of an extracellular matrix, and the like.

The vascular endothelial cell that can be combined with the cell or the like of the present invention or the pharmaceutical composition of the present invention is not particularly limited, and in one embodiment, the vascular endothelial cell is a primary vascular endothelial cell or a vascular endothelial cell obtained by inducing differentiation of a pluripotent stem cell or the like. The vascular endothelial cell that can be used in combination with the cell or the like of the present invention or the pharmaceutical composition of the present invention is not particularly limited, and in one embodiment, the vascular endothelial cell is a primary vascular endothelial cell, or a vascular endothelial cell obtained by inducing differentiation of a pluripotent stem cell or the like. In the treatment method of the present invention, when the cell or the like of the present invention and a vascular endothelial cell are used in combination, the vascular endothelial cell can be administered simultaneously with the administration of the cell or the like of the present invention, or before the administration or after the administration of the cell or the like of the present invention.

The term "primary vascular endothelial cell" means a vascular endothelial cell directly collected from an individual organism, or a primary cultured cell or a passaged cell obtained by culturing and proliferating the vascular endothelial cell in vitro. The primary vascular endothelial cell is not particularly limited, and in an embodiment, the primary vascular endothelial cell is a human primary vascular endothelial cell. As the human primary vascular endothelial cell, for example, it is desirable to use a primary vascular endothelial cell from a patient himself/herself who is a human, or an individual who is a transplant recipient having the same or substantially the same human leukocyte antigen (HLA) genotype as that of the patient from the viewpoint of avoiding rejection reaction. Here, the term "substantially the same" means that the HLA genotypes match each other to the extent that an immune reaction to the transplanted vascular endothelial cell can be suppressed by an immunosuppressive agent, and for example, such a vascular endothelial cell is a vascular endothelial cell having an HLA type in which 3 loci of HLA-A, HLA-B, and HLA-DR or 4 loci consisting of these 3 loci and HLA-C are matched. In one embodiment, the vascular endothelial cell that can be used in combination with or combined with the cell or the like of the present invention or the pharmaceutical composition of the present invention is a human primary vascular endothelial cell.

The "vascular endothelial cell obtained by inducing differentiation of a pluripotent stem cell or the like" that can be combined with the cell or the like of the present invention or the pharmaceutical composition of the present invention is not particularly limited, and a vascular endothelial cell produced by the method disclosed in, for example, Ikuno T et al., Pros One, (2019), 12: e0173271 or Cho SW et al., Circulation, (2007), 116: 2409-2419 can be used. In one embodiment, the vascular endothelial cell that can be used in combination with or combined with the cell or the like of the present invention or the pharmaceutical composition of the present invention is a vascular endothelial cell obtained by inducing differentiation of a human pluripotent stem cell or the like.

Specific Examples referred to in order to obtain a further understanding of the invention will be provided here, but these are for an illustrative purpose and do not limit the invention.

### EXAMPLES

### Example 1: Induction of differentiation of ES cell into early mesodermal cell

230 µL of Matrigel (registered trademark) Human ES Cell Qualified Matrix (Corning, 354277) was added to 25 mL of DMEM/F12 medium (Nacalai Tesque, 11581-15), and 1.5 mL was added to each of 3 wells on a 6-well plate (Iwaki, 3810-006) and allowed to stand at room temperature for 3 hours to prepare a Matrigel-coated plate. A human ES cell (National Center for Child Health and Development, SEES6) suspended in an mTeSR1 medium (STEMCELL Technologies, ST-85850) supplemented with 10 µM Y-27632 (Nacalai Tesque, 08945), a ROCK inhibitor, was seeded in the 3 wells on the Matrigel-coated plate, each at 5.0 × 10⁵ cells/well, and cultured at 37°C in an atmosphere of 5% CO₂ for 24 hours. Thereafter, the supernatant was removed, and 3.5 mL of STEMdiff Mesoderm Induction Medium (STEMCELL Technologies, 05220) was added to each of the wells. After 24 hours, the supernatant was removed again, and 3.5 mL of STEMdiff Mesoderm Induction Medium was added to each of the wells and further cultured at 37°C in an atmosphere of 5% CO₂ for 24 hours. The supernatant was removed, washing with PBS was carried out, 1 mL of TrypLE Express (Thermo Fisher Scientific, 12604013) was added to each of the wells in order to detach the cells from the plate, and the resultant was allowed to stand on a 37°C plate for 5 minutes. Next, 5 mL of the spheroid formation medium (see below) was added to each of the wells to recover detached cells. Whether the recovered cell is differentiated into an early mesodermal cell was confirmed by flow cytometry. Specifically, PDGFRα and APLNR were selected as cell surface markers of an early mesodermal cell, and a BV421 Anti-Human CD140α (PDGFRα) antibody (BD, 562799) and an APC Anti-Human APJ antibody (R&D Systems, FAB8561A-025) were used as antibodies against each cell surface marker, respectively. 4 mL of the recovered cell suspension was added, and centrifuged under conditions of 300 g, room temperature, and 5 minutes. The supernatant was removed, 1 mL of 2% FBS (Sigma-Aldrich)-containing PBS was added, and the resultant was centrifuged again under conditions of 300 g, room temperature, and 5 minutes. The supernatant was removed, 100 µL of 2% FBS-containing PBS was added, the cells were suspended, then 5 µL of FcR Blocking Reagent (Miltenyi Biotec, 130-059-901) was added, and the resultant was allowed to stand on ice for 30 minutes. To 20 µL of the cell suspension after standing on ice for 30 minutes, 5 µL of a BV421 Anti-Human CD140α antibody and 10 µL of an APC Anti-Human APJ antibody were added, and the resultant was allowed to react on ice for 20 minutes. After the reaction, washing with 2% FB S-containing PBS was carried out twice. In addition, the supernatant of human ES cells (SEES6s) that had been cultured by using mTeSR1 medium on the Matrigel-coated plate was also removed, washing with PBS was carried out, and 1 mL of TrypLE Express was added in order to detach the cells from the plate, and the resultant was allowed to stand on a 37°C plate for 5 minutes. Next, 5 mL of the mTeSR1 medium was added to recover detached cells. 4 mL of the suspension of the recovered cells was centrifuged under conditions of 300 g, room temperature, and 5 minutes. The supernatant was removed, 150 µL of 2% FBS-containing PBS was added, and further, 5 µL of FcR Blocking Reagent was added, and the resultant was allowed to stand on ice for 20 minutes. After centrifugation under conditions of 300 g, room temperature, and 5 minutes, the supernatant was removed, and 60 µL of 2% FBS-containing PBS was added to suspend the cells. To 20 µL of the cell suspension, 5 µL of a BV421 Anti-Human CD140α antibody and 10 µL of an APC Anti-Human APJ antibody were added, and the resultant was allowed to react on ice for 20 minutes. After the reaction, washing with 2% FBS-containing PBS was carried out twice. As a result of analyzing the two cells reacted with the two antibodies by using Attune NxT Flow Cytometer (Invitrogen), almost no PDGFRα (+) and APLNR(+) cells were present in the human ES cell before culture using STEMdiff Mesoderm Induction Medium (Fig. 1, left), whereas 55% or more of the cells after 48 hours of culture with STEMdiff Mesoderm Induction Medium exhibited PDGFRα(+) and APLNR(+) (Fig. 1, right). The results indicate that culture using STEMdiff Mesoderm Induction Medium enhanced induction differentiation of the ES cell into an early mesodermal cell. The cell obtained by culturing the ES cell using STEMdiff Mesoderm Induction Medium is hereinafter referred to as an "early mesodermal cell".

### [Spheroid formation medium]

A spheroid formation medium for forming a spheroid of an early mesodermal cell was prepared by improving a method disclosed in the literature (Vodyanik MA et al., Cell Stem Cell, (2010), 7: 718-729). Specifically, each was mixed to be 40% MegaCell Dulbecco's Modified Eagle's Medium (Sigma-Aldrich, M3942), 25% StemSpan SFEM (STEMCELL Technologies, 09650), 25% Human Endothelial SFM (Thermo Fisher Scientific, 11111044), and 10% BIT 9500 Serum Substitute (STEMCELL Technologies, 09500). Further, the spheroid formation medium was prepared by adding each material thereto so as to have 1% GlutaMax (Thermo Fisher Scientific, 35050061), 0.1% Ex-CYTE (registered trademark) NZ Growth Enhancement Media Supplement (Merck, 81150N), 100 µM Monothioglycerol (FUJIFILM Wako Pure Chemical Corporation, 195-15791), 50 µg/mL L-ascorbic acid 2-phosphate sesquimagnesium salt hydrate (Sigma-Aldrich, A8960), and 20 ng/mL Animal-free Recombinant Human FGF basic-TS (Proteintech, HZ-1285).

### Example 2: Formation of spheroid from early mesodermal cell

10 mL of the spheroid formation medium was added to 1.7 × 10⁵ cells of the early mesodermal cell obtained in Example 1, which was then seeded on an EZSPHERE (registered trademark) dish (Iwaki, 11-0434) and cultured at 37°C in an atmosphere of 5% CO₂ and 5% O₂. 10 mL of the spheroid formation medium was gradually added 4 days after the start of culture. 10 mL of each culture supernatant was removed 6, 8, and 11 days after of the start of culture, and 10 mL of a newly spheroid formation medium was gradually added. The culture solution including the spheroid was passed through a Falcon (registered trademark) 100 µm cell strainer (Corning, 352360) 12 days after the start of culture to recover a spheroid having a size of 100 µm or more.

### Example 3: Induction of differentiation of spheroid into pericyte-like cell

2 mL of PBS including a final concentration of 3 µg/mL Fibronectin (Corning, 356008) and 10 µg/mL Human Type1 Collagen (Kyowa Pharma Chemical Co., Y-1) was added to a Falcon (registered trademark) 60 mm cell culture dishes (Corning, 353002) and allowed to stand at 37°C for 3 hours. All the spheroids recovered in Example 2 were suspended in a pericyte differentiation induction medium (see below), seeded on two of the dishes, and cultured at 37°C in an atmosphere of 5% CO₂ and 5% O₂. Monolayer cell proliferation is observed on the bottom of the dish 3 days after the start of culture. At this point, the culture supernatant was removed, washing with PBS was carried out, then 1 mL of Accutase (Innovative Cell Technologies, AT104) was added to the dishes in order to detach the cells, and the resultant was allowed to stand on a 37°C plate for 5 minutes. 4 mL of the pericyte growth medium (see below) was added to the dishes, and the cell suspension was recovered and centrifuged under conditions of 300 g, room temperature, and 5 minutes. The supernatant was removed, 2 mL of a pericyte growth medium was added, and the cells were seeded at 2 × 10⁵ cells/dish on 60-mm collagen-coated dishes (Corning, 354401) and cultured at 37°C in an atmosphere of 5% CO₂ and 5% O₂. The cells cultured in the pericyte differentiation induction medium followed by the pericyte growth medium and recovered are pericyte-like cells and used in the following experiments.

### [Pericyte differentiation induction medium]

The composition is as follows:
· 50% Stemline (registered trademark) II Hematopoietic Stem Cell Expansion Medium (Sigma-Aldrich, S0192)
· 50% Human Endothelial SFM
· 1% GlutaMax
· 0.05% Ex-CYTE NZ Growth Enhancement Media Supplement
· 100 µM Monothioglycerol
· 10 ng/mL Animal-free Recombinant Human FGF basic-TS
· 50 ng/mL Recombinant Human PDGF-BB Protein (R&D Systems, 220-BB)

### [Pericyte growth medium]

The composition is as follows:
· 80% MegaCell Dulbecco's Modified Eagle's Medium
· 20% FBS
· 1% GlutaMax
· 1% MEM Non-essential Amino Acid Solution (Sigma-Aldrich, M7145)
· 1% Penicillin-Streptmycin (Sigma-Aldrich, P0781)
· 100 µM 2-Mercaptethanol (Thermo Fisher Scientific, 21985023)
· 5 ng/mL Animal-free Recombinant Human FGF basic-TS

### Example 4: Selection of CD56(+) pericyte-like cell and CD56(-) pericyte-like cell

The pericyte-like cells obtained in Example 3 were seeded in five 10-cm collagen-coated dishes (Corning, 356450) at 2 × 10⁵ cells/dish in the presence of the pericyte growth medium and cultured at 37°C in an atmosphere of 5% CO₂ and 5% O₂. After 4 days, the supernatant was removed, washing with PBS was carried, then 2 mL of Accutase was added to the dish in order to detach the cells, and the resultant was allowed to stand on a 37°C plate for 5 minutes. 8 mL of the pericyte growth medium was added to each of the dishes, and the cell suspension was recovered and centrifuged under conditions of 300 g, room temperature, and 5 minutes. The supernatant was removed, and 10 mL of the pericyte growth medium was added. From this cell suspension, 1 × 10⁷ cells were placed in another tube and centrifuged under conditions of 300 g, room temperature, and 5 minutes. The supernatant was removed, 100 µL of PBS was added, and further, 20 µL of FcR Blocking Reagent was added, and the resultant was allowed to stand on ice for 15 minutes. Subsequently, 30 µL of an Anti-CD56 PE antibody (Miltenyi Biotec, 130-090-755) was added, and the resultant was allowed to stand on ice for 20 minutes. Washing with PBS was carried out twice and then suspension in PBS was carried out again, and a CD56(+) pericyte-like cell and a CD56(-) pericyte-like cell were separated by using a cell sorter, SH800S (Sony) (Fig. 2, upper). Each of the separated cells was suspended in the pericyte growth medium, seeded on a 10 cm collagen-coated dish, and cultured.

### Example 5: Quantitation of VEGF expression level in CD56(+) pericyte-like cell and CD56(-) pericyte-like cell

Each of the 3 × 10⁵ CD56(-) pericyte-like cells and 3 × 10⁵ CD56(+) pericyte-like cells separated in Example 4 was cultured on a 10 cm collagen-coated dish using 10 mL of a pericyte growth medium, and the culture supernatant was recovered after 3 days. An immunoassay kit (Human VEGF immunoassay kit, PerkinElmer, AL201C) was used to measure the VEGF concentration in the recovered culture supernatant. The measurement of the VEGF concentration followed the protocol attached to the kit. As a result, the expression level of VEGF in the CD56(-) pericyte-like cells was significantly higher than that in the CD56(+) pericyte-like cells (Fig. 2, lower). As a statistical analysis test, a t-test was carried out between two groups.

### Example 6: Surface antigen analysis of CD56(-) pericyte-like cell

The CD56(-) pericyte-like cells separated in Example 4 were cultured in the pericyte growth medium, and the expression of multiple cell surface antigens was examined using an Attune NxT Flow Cytometer as follows.

Antibodies against cell surface antigens used were a BV421 Anti-Human CD73 antibody (BD, 562430), PE Anti-Human CD140b antibody (BD, 558821), BB515 Anti-Human CD146 antibody (BD, 564644), APC Anti-Human NG2 antibody (R&D Systems, FAB2585A), PE Anti-Human HLA-ABC antibody (BD, 557349), BV421 Anti-Human HLA-DR,DP,DQ antibody (BD, 564244), BV421 Anti-Human CD105 antibody (BD, 563920), BV421 Anti-Human CD90 antibody (BioLegend, 328122), APC Anti-Human CD44 antibody (BioLegend, 338806), APC Anti-Human CD13 antibody (BioLegend, 301706), an APC Anti-Human CD31 antibody (BioLegend, 303116), and an APC Anti-Human CD45 antibody (BioLegend, 304012).

8 1.5 mL tubes (Eppendorf, 0030120.086) were prepared, and 3 × 10⁵ CD56(-) pericyte-like cells were placed in each tube, to which 500 µL of 2% FBS-containing PBS was added, washing was carried out once, and then the resultant was suspended and added to 30 µL of 2% FBS-containing PBS. 3 µL of FcR Blocking Reagent was added in the same step as in Example 4, and the tubes were allowed to stand on ice for 15 minutes. Subsequently, one of the antibodies or several antibodies having no overlapping fluorescent wavelengths were added to each tube in an amount recommended by the manufacturer, and the tubes were allowed to stand on ice for 20 minutes. Next, 500 µL of 2% FBS-containing PBS was added to each tube and washed twice, and then the cells were suspended again in 500 µL of 2% FB S-containing PB S and analyzed using an Attune NxT Flow Cytometer. As a result, the CD56(-) pericyte-like cells separated in Example 4 were positive for the expression of CD13, CD44, CD73, CD90, CD105, CD140b, CD146, NG2, and HLA-ABC (Class I) (but some cells were negative for CD90), while they were negative for the expression of CD31, CD45, and HLA-DR, DP, and DQ (Class II). That is, CD56(-) pericyte-like cells induced from ES cells via early mesodermal cells in Examples 1 to 3 and selected in Example 4 were found to express surface antigens similar to primary pericytes derived from the living body (Cathery M et al., STEM CELLS, (2018), 36: 1295-1310) (Fig. 3).

### Example 7: Growth curve of CD56(-) pericyte-like cell

4.8 × 10⁵ CD56(-) pericyte-like cells were cultured by using 10 mL of a pericyte growth medium on a 10 cm collagen-coated dish. After several days, once the cells were confirmed to have proliferated to a confluent state, the supernatant was removed, washing with PBS was carried out, then 2 mL of Accutase was added, and the resultant was allowed to stand on a 37°C plate for 5 minutes. 8 mL of the pericyte growth medium was added to the dish, and the cell suspension was recovered and centrifuged under conditions of 300 g, room temperature, and 5 minutes. The supernatant was removed, 3 mL of the pericyte growth medium was added, and the number of cells in the suspension was counted. Thereafter, 1.5 × 10⁵ to 3.0 × 10⁵ CD56(-) pericyte-like cells were subcultured by repeating the procedure of culturing on a new 10 cm collagen-coated dish using 10 mL of a pericyte growth medium. As a control, a human tissue -derived primary pericyte was used. The human tissue-derived primary pericyte was obtained by the following method: That is, 3 × 10⁴ primary pericytes were cultured by using 5 mL of a pericyte growth medium on a 60 mm collagen-coated dish. After several days, once the cells were confirmed to have proliferated to a confluent state, the supernatant was removed, washing with PBS was carried out, then 1 mL of Accutase was added, and the resultant was allowed to stand on a 37°C plate for 5 minutes. 4 mL of the pericyte growth medium was added to the dish, and the cell suspension was recovered and centrifuged under conditions of 300 g, room temperature, and 5 minutes. The supernatant was removed, 3 mL of the pericyte growth medium was added, and the number of cells in the suspension was counted. Thereafter, 1.5 × 10⁵ to 5.0 × 10⁵ primary pericytes were subcultured by repeating the procedure of culturing on a 10 cm collagen-coated dish using 10 mL of a pericyte growth medium. For CD56(-) pericyte-like cells and primary pericytes, the number of passage days was plotted on the horizontal axis and the cumulative number of cell divisions on the vertical axis (Fig. 4). The CD56(-) pericyte-like cells showed higher proliferation ability than the primary pericytes.

### Example 8: Qualitative evaluation of angiogenic potential of CD56(-) pericyte-like cell

Human Umbilical Vein Endothelial Cells (HUVEC, PromoCell, C-12200) were cultured at 37°C in an atmosphere of 5% CO₂ by using an endothelial cell growth medium (PromoCell, C-22111). At the same time, the CD56(-) pericyte-like cell selected by the same procedure as in Example 4 and the CD56(+) pericyte-like cell as a control were each cultured by using a pericyte growth medium on a 10 cm collagen-coated dish. Once each of the three cells was confirmed to have proliferated to a confluent state, the cell suspension was recovered by the same procedure as in Example 7 (however, an endothelial cell growth medium was used for the suspension of the HUVEC) and centrifuged under conditions of 300 g, room temperature, and 5 minutes to remove the supernatant, and then the resultant was suspended in an endothelial cell growth medium for the HUVEC, and in a pericyte growth medium for the two pericyte-like cells, and the number of cells in the suspension was counted. The HUVEC was added to 9 1.5 mL tubes at 5.5 × 10⁵ cells per tube. To 3 tubes thereof the CD56(-) pericyte-like cell was further added and to another 3 tubes thereof the CD56(+) pericyte-like cell was added, each at 5.5 × 10⁵ cells, and admixed. Each tube was centrifuged under conditions of 300 g, 4°C, and 5 minutes to remove the supernatant, further, washing with PBS was carried out once, and then the tube was centrifuged again under the same conditions to remove the supernatant. Next, 400 µL of an extracellular matrix (Matrigel (registered trademark) Growth factor reduced, Corning, 356231, hereinafter referred to as "Matrigel") was added to each tube, admixed on ice, and then Matrigel including the cell was aspirated with a syringe equipped with a 25 gauge needle.

To 79 mL of physiological saline (Otsuka Pharmaceutical Factory, Inc.), 3 mL of Domitor (Nippon Zenyaku Kogyo Co., Ltd.), 8 mL of Dormicum Injection (Maruishi Pharmaceutical Co., Ltd.), and 10 mL of Betorfal (Meiji Seika Pharma Co., Ltd.) were added to prepare a triple mixed anesthetic solution. 300 µL of the triple mixed anesthetic solution was administered to the abdominal cavity of each of 5 NOG mice (NOD.Cg-Prkdc^{scid}I12rg^{tm1Sug}/ShiJic mice, In-Vivo Science). Thereafter, the whole amount of Matrigel including any of the HUVEC alone, the HUVEC and the CD56(-) pericyte-like cell (CD56(-)/HUVEC), and the HUVEC and the CD56(+) pericyte-like cell (CD56(+)/HUVEC) prepared was subcutaneously administered to each of the NOG mice under anesthesia. After 15 days, the administered Matrigel was recovered from each mouse and photographed. As a result, Matrigel including the CD56(-) pericyte-like cell showed more enhanced angiogenesis than Matrigel including the CD56(+) pericyte-like cell. On the other hand, no angiogenesis was observed in Matrigel including HUVEC alone (Fig. 5).

### Example 9: Quantitative evaluation of angiogenesis exhibited by CD56(-) pericyte-like cell

Each Matrigel recovered by the procedure of Example 8 was placed in a 2 mL tube (Eppendorf, 0030120.094) and cut several times with dissecting scissors. One stainless steel bead (Qiagen, 69989) was added thereto, and 350 µL of 0.1% Brij (registered trademark) L23 solution (Sigma-Aldrich, B4184) diluted with water was further added. Matrigel was crushed by using TissueLyzer II (Qiagen, 85300) and centrifuged under conditions of 10,000 g, 4°C, and 5 minutes, and then the supernatant was recovered. The concentration of hemoglobin in the recovered supernatant was measured by using QuantiChrom Hemoglobin Assay Kit (BioAssay Systems, DIHB-250). The measurement method followed the product protocol. As a statistical analysis test, a t-test was carried out between two groups of the HUVEC and the CD56(+) pericyte-like cell (CD56(+)/HUVEC), and the HUVEC and the CD56(-) pericyte-like cell (CD56(-)/HUVEC). As a result, Matrigel including the CD56(-) pericyte-like cell exhibited significantly higher concentration of hemoglobin than Matrigel including the CD56(+) pericyte-like cell (Fig. 6).

### INDUSTRIAL APPLICABILITY

A VEGF-highly expressing pericyte-like cell can be produced by the production method of the present invention. The VEGF-highly expressing pericyte-like cell obtained by the production method of the present invention has a higher cell proliferation ability than a primary pericyte available in the past and excellent angiogenic action and can be used for an angiogenic therapy for critical lower limb ischemia or the like.

## Claims

1. A VEGF-highly expressing pericyte-like cell obtained by a production method comprising a step of selecting a CD56(-) pericyte-like cell from a population including a pericyte-like cell obtained by inducing differentiation of a pluripotent stem cell.

2. The VEGF-highly expressing pericyte-like cell according to claim 1, which is obtained by a production method comprising:
(a) a step of differentiating a pluripotent stem cell into an early mesodermal cell;
(b) a step of differentiating the early mesodermal cell obtained in step (a) into a pericyte-like cell; and
(c) a step of selecting a CD56(-) pericyte-like cell from a population including the pericyte-like cell.

3. The VEGF-highly expressing pericyte-like cell according to claim 1, which is obtained by a production method comprising:
(a) a step of differentiating a pluripotent stem cell into an early mesodermal cell;
(b-1) a step of forming a spheroid of the early mesodermal cell obtained in step (a);
(b-2) a step of differentiating the early mesodermal cell forming the spheroid into a pericyte-like cell; and
(c) a step of selecting a CD56(-) pericyte-like cell from a population including the pericyte-like cell.

4. The VEGF-highly expressing pericyte-like cell according to claim 1, which is obtained by a production method comprising:
(a) a step of differentiating a pluripotent stem cell into an early mesodermal cell;
(b-1') a step of forming a spheroid of the early mesodermal cell obtained in step (a) in a culture vessel comprising a plurality of mortar-shaped fine wells on a culture surface without a gap;
(b-2) a step of differentiating the early mesodermal cell forming the spheroid into a pericyte-like cell; and
(c) a step of selecting a CD56(-) pericyte-like cell from a population including the pericyte-like cell.

5. The VEGF-highly expressing pericyte-like cell according to any one of claims 1 to 4, wherein the pluripotent stem cell is a human pluripotent stem cell.

6. The VEGF-highly expressing pericyte-like cell according to any one of claims 1 to 5, wherein the pluripotent stem cell is an ES cell or an iPS cell.

7. A population of VEGF-highly expressing pericyte-like cells, which is selected using a cell surface marker of CD56(-) from a population including a pericyte-like cell obtained by inducing differentiation of a pluripotent stem cell.

8. The population of VEGF-highly expressing pericyte-like cells according to claim 7, which is selected using a cell surface marker of CD56(-) from a population including a pericyte-like cell obtained by inducing differentiation by a method comprising:
(a) a step of differentiating a pluripotent stem cell into an early mesodermal cell; and
(b) a step of differentiating the early mesodermal cell obtained in step (a) into a pericyte.

9. The population of VEGF-highly expressing pericyte-like cells according to claim 7, which is selected using a cell surface marker of CD56(-) from a population including a pericyte-like cell obtained by inducing differentiation by a method comprising:
(a) a step of differentiating a pluripotent stem cell into an early mesodermal cell;
(b-1) a step of forming a spheroid of the early mesodermal cell obtained in step (a); and
(b-2) a step of differentiating the early mesodermal cell forming the spheroid into a pericyte-like cell.

10. The population of VEGF-highly expressing pericyte-like cells according to claim 7, which is selected using a cell surface marker of CD56(-) from a population including a pericyte-like cell obtained by inducing differentiation by a method comprising:
(a) a step of differentiating a pluripotent stem cell into an early mesodermal cell;
(b-1') a step of forming a spheroid of the early mesodermal cell obtained in step (a) in a culture vessel comprising a plurality of mortar-shaped fine wells on a culture surface without a gap; and
(b-2) a step of differentiating the early mesodermal cell forming the spheroid into a pericyte-like cell.

11. The population of VEGF-highly expressing pericyte-like cells according to any one of claims 7 to 10, wherein the pluripotent stem cell is a human pluripotent stem cell.

12. The population of VEGF-highly expressing pericyte-like cells according to any one of claims 7 to 11, wherein the pluripotent stem cell is an ES cell or an iPS cell.

13. A pharmaceutical composition for an angiogenic therapy, comprising the VEGF-highly expressing pericyte-like cell according to any one of claims 1 to 6.

14. The pharmaceutical composition according to claim 13, wherein the angiogenic therapy is a treatment for critical lower limb ischemia.

15. The pharmaceutical composition according to claim 13 or claim 14, wherein the pharmaceutical composition is used in combination with a vascular endothelial cell.

16. A pharmaceutical composition for an angiogenic therapy, comprising a combination of the VEGF-highly expressing pericyte-like cell according to any one of claims 1 to 6 and a vascular endothelial cell.

17. The pharmaceutical composition according to claim 16, wherein the angiogenic therapy is a treatment for critical lower limb ischemia.

18. The VEGF-highly expressing pericyte-like cell according to any one of claims 1 to 6 for use in an angiogenic therapy.

19. The VEGF-highly expressing pericyte-like cell according to any one of claims 1 to 6 for use in treatment for critical lower limb ischemia.

20. A pharmaceutical composition for an angiogenic therapy, comprising the population of VEGF-highly expressing pericyte-like cells according to any one of claims 7 to 12.

21. The pharmaceutical composition according to claim 20, wherein the angiogenic therapy is a treatment for critical lower limb ischemia.

22. The pharmaceutical composition according to claim 20 or 21, wherein the pharmaceutical composition is used in combination with a vascular endothelial cell.

23. A pharmaceutical composition for an angiogenic therapy, comprising a combination of the population of VEGF-highly expressing pericyte-like cells according to any one of claims 7 to 12 and a vascular endothelial cell.

24. The pharmaceutical composition according to claim 23, wherein the angiogenic therapy is a treatment for critical lower limb ischemia.

25. The population of VEGF-highly expressing pericyte-like cells according to any one of claims 7 to 12 for use in an angiogenic therapy.

26. The population of VEGF-highly expressing pericyte-like cells according to any one of claims 7 to 12 for use in treatment for critical lower limb ischemia.
